**Europäisches Patentamt**

**European Patent Office**

**Office européen des brevets**

(19)

(11) Publication number: **0 233 109**
A2

(12) # EUROPEAN PATENT APPLICATION

(21) Application number: **87400148.0**

(22) Date of filing: **22.01.87**

(51) Int. Cl.⁴: **A 61 L 9/12, A 01 M 13/00**

(30) Priority: **23.01.86 JP 8315/86 U**

(43) Date of publication of application: **19.08.87 Bulletin 87/34**

(84) Designated Contracting States: **DE FR IT**

(71) Applicant: **Shin-Etsu Chemical Co., Ltd., 6-1, Ohtemachi 2-chome, Chiyoda-ku Tokyo 100 (JP)**

(72) Inventor: **Aiba, Noboru, 3-2-21, Kitahoncho, Joetsu-shi Niigata-ken (JP)**
Inventor: **Nagura, Shigehiro, 2-1-4-7, Minato-cho, Joetsu-shi Niigata-ken (JP)**
Inventor: **Tezuka, Haruya, 804-85, Otani, Omiya-shi Saitama-ken (JP)**
Inventor: **Yamamoto, Akira, 2-14-45, Gochi, Joetsu-shi Niigata-ken (JP)**

(74) Representative: **Armengaud Ainé, Alain et al, Cabinet ARMENGAUD AINE 3 Avenue Bugeaud, F-75116 Paris (FR)**

(54) **A vapor dispenser.**

(57) A tubular plastic-made vapor dispenser for sustainedly releasing vapor of an insectan sex pheromone is proposed to be used in the pest control in the fields. The tubular vapor dispenser has specified length, inner diameter and wall thickness and is supplied in a plastically deformed configuration forming at least one loop. This unique configuration of the tubular vapor dispenser ensures remarkably improved uniformity in the rate of vapor release over a long period of time.

0233109

## A VAPOR DISPENSER

## BACKGROUND OF THE INVENTION

The present invention relates to a vapor dispenser or, more particularly, to a dispenser for sustainedly releasing vapor of a chemical substance capable of exhibiting very distinguished biological effects, such as an insectan sex pheromone, even when the vapor of the substance is contained in the atmospheric air in a very low or trace concentration.

As is known, various kinds of vaporizable chemical substances exhibit very distinguished biological effects when the vapor thereof is contained in the atmospheric air in a very low or trace concentration including perfumes, insecticides, sex pheromones of insects and so on. It is highly desirable that the atmospheric air of the sites where the effects of the vaporizable substance should be exhibited is filled with the vapor of the substance sustainedly in a low but controlled concentration, especially, in consideration of economy when the vaporizable substance is expensive as is the case with most of synthetically prepared sex pheromones of insects recently used for controlling the population of insectan pests in the fields such as farms, orchards and forests. In this regard, it is a conventional practice that the vaporizable substance is contained in a body which serves as a dispenser of the substancer to sustainedly release the vapor thereof into the atmosphere. A large number of such dispensers are distributed over the field and

-sustainedly release the vapor to maintain the desired concentration of the substance over an area. Various types of vapor dispensers have been proposed and actually tested in the prior art.

For example, U.S. Patent 4,600,146 discloses a plastic-made tubular dispenser body containing the vaporizable substance, e.g. insectan sex pheromone, in the capillary bore thereof and the vaporizable substance permeates the tube walls and is released to the atmosphere off the outer surface. The tubular dispenser should preferably be bonded side by side in the longitudinal direction with a metal wire having plastic deformability so that the dispenser body can retain any form as bent to ensure convenience and reliability in fixedly installing the dispensers having an appropriate deformed configuration in desired positions, e.g. on the twigs in an orchard.

The above mentioned wire-bonded tubular dispensers have, however, several disadvantages. For example, the costs for the production thereof are necessarily high including the cost for the wire bonding works and the increased cost for filling the capillary bore with the vaporizable substance in addition to the somewhat time-consuming works for installing the freshly prepared dispensers on and dismounting them after exhaustion of the vaporizable substance or after lapse of the desired period from the positions in which they had been kept for a length of time. When such wire-bonded tubular dispensers filled with an insectan sex pheromone are used by hanging on the twigs of trees and the like, moreover, the efficiency of the pruning works of the twigs may be greatly decreased due to possible damages on the edges of scissors or cutting blades by hitting the metal wires unless the pruning works are performed after complete removal of the dispensers from the trees.

## SUMMARY OF THE INVENTION

The object of the present invention is therefore to provide a vapor dispenser for sustainedly releasing vapor of a vaporizable chemical substance free from the above described disadvantages and problems in the vapor dispensers of prior art.

Thus, the vapor dispenser of the present invention is a tube having a length of at least 120 mm, an inner diameter in the range from 0.4 to 3.0 mm and a wall thickness in the range from 0.1 to 2.0 mm made of a plastic and filled in the bore with at least 40 mg of an insectan sex pheromone compound capable of permeating the wall of the tube and of being released to the atmosphere off the outer surface of the tube, both ends of the tube being air-tightly sealed and the tube being in a curved configuration forming at least one loop.

It is preferable that the tube is made of a polyethylene and the length of a chord passing through the center of the loop is at least-20-mm as measured between oppositely facing inward portions of the generating lines of the tube surface.

## BRIEF DESCRIPTION OF THE DRAWING

FIGURE 1 is a plan view of a vapor dispenser according to the invention forming a single loop.

FIGURE 2 is a cross sectional view of a vapor dispenser according to the invention forming a single loop as cut along the axial line of the tube.

FIGURE 3 is a plan view of another vapor dispenser according to the invention forming two loops.

## DETAILED DESCRIPTION OF THE PREFERRED EMBODIMENTS

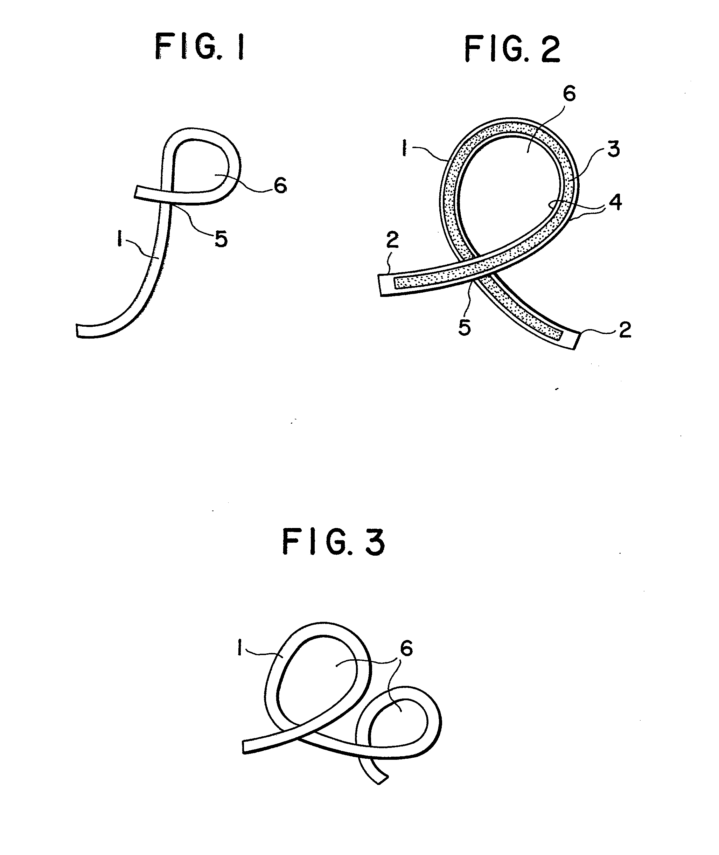

In the following, the vapor dispenser of the invention is described in more detail with reference to the accompanying drawing. FIGURES 1 and 2 illustrate a vapor dispenser of the invention forming a single loop by a plan view and by an axial cross sectional view, respectively. The tubular body 1 is made of a plastic resin, which is preferably a polyethylene, and should have an inner diameter in the range from 0.4 to 3.0 mm and a thickness of the wall 4 in the range from 0.1 to 2.0 mm. The tube 1 is filled in the bore with a vaporizable insectan pheromone compound 3 in a liquid form and air-tightly sealed at both tube ends 2,2 by a suitable sealing means such as welding, plugging with a sealant and so on.

—5—

0233109

When the inner diameter of the tube is too small, difficulties are encountered in molding tubes having a capillary bore of so small diameter in addition to the difficulty in filling the capillary bore with the pheromone compound. Although a larger inner diameter of the tubular body 1 is desirable from the standpoint of filling the bore with a larger amount of the pheromone compound, a too large inner diameter of the bore is not always advantageous because the pheromone compound filling the bore in a too large amount is subject to denaturation in the lapse of time of, for example, one year or longer by direct sunlight or by oxidation with the atmospheric air so that no constancy is ensured in the rate of vapor release over a length of time. The wall thickness is a factor profoundly influencing the rate of vapor release through the tube walls although plastic tubes having a wall thinner than 0.1 mm can hardly be shaped by a conventional molding means while a too large wall thickness causes an unduly low rate of vapor release in addition to an economical disadvantage and difficulty in forming a looped configuration. The tube 1 should have a length of at least 120 mm since no loop can be formed with a shorter tube or only a too small loop can be formed. It is optional that the tubular body is partitioned into two or more sectiones by collapsingly welding or by providing partitioning walls at one or more positions without particularly affecting the performance of the inventive vapor dispenser.

The amount of the pheromone compound 3 filling the tubular bore should be at least 40 mg in order to ensure a duration of sustained vapor release as long as desired although the exact amount thereof should depend on various parameters including the kind of the plastic forming the tube wall 4, wall thickness, permeability of the pheromone compound 3 through the tube walls 4, desired concentration of the vapor of the pheromone compound in the atmosphere and so on in addition to the above mentioned duration of service. It may be too much to say that the pheromone compound 3 must have permeability through the walls 4 of the tube 1. In other words, the plastic material forming the tube 1 should be appropriately selected depending on the nature of the pheromone compound 3 which should permeate through the tube walls 4.

It is essential in the inventive vapor dispenser that the tube 1 is deformed in a curved configuration forming at least one loop 6 to intersect with itself at the point 5. It is of course optional that the tube in the thus curved configuration may form two or more loops as is illustrated in FIGURE 3. Though not particularly limitative, the loop thus formed should preferably have such a size that the length of a chord is at least 20 mm as measured between oppositely facing inward portions of the generating lines of the tube surface. The above mentioned loop-forming configuration of the tube 1 is desirable in order to ensure good constancy of the rate of sustained vapor release over a long range of service period. In addition, the loop-forming configuration of the tubular dispenser body has an additional but secondary advantage that a large number of the dispensers as supplied can be efficiently put into the spots such as tree twigs without further modification of the configuration. Once such a tubular dispenser of a looped configuration is hung on a twig, the dispenser can be reliably held thereon withstanding a stormy weather of a wind velocity of at least 15 meters/second without falling.

The tubular dispensers 1 forming a single loop 6 as illustrated in FIGURES 1 and 2 each have legs with the tube ends 2,2 extending from the intersection 5. It is, however, optional that one of the tube ends 2,2 is in contact with the intersection 5 without forming an extending leg in a configuration something like a Greek letter ρ. The two legs of the tube 1 intersecting at the intersection 5 may be merely contacting with each other or may be bonded together by welding or using an adhesive. At any rate, it is desirable that the gap at the intersection 5 between the two legs of the tube 1 is not larger than 3 times of the outer diameter of the tube 1 to retain an appearance of a looped configuration as a whole.

The type of the plastic resin forming the tubular dispenser body 1 of the invention is not particularly limitative provided that the resin has sufficient plasticity in order that the resin can be shaped into a tubular form by a conventional means such as extrusion molding and further the thus shaped tube can be deformed into a loop-forming configuration. Suitable plastic resins include polyolefins, e.g. polyethylenes and polypropylenes, polymers of acrylic and methacrylic esters, polyamide resins, polyesters, cellulose derivatives, polyorganosiloxanes and the like, of which polyethylenes are preferred. It is of course optional that the resin is compounded with various kinds of known additives such as plasticizers, lubricants, stabilizers, coloring agents, ultraviolet absorbers and the like according to need. The resin or resin compound may be admixed with a crosslinking agent and the tube after molding is brought under a condition for forming crosslinks into a three-dimensional network structure.

According to the results of the investigations in recent years, the concentration of the pheromone vapor in the atmosphere is a more important factor for the efficiency of disturbing the intersexual communication by the pheromone between different sexes of insects than the number of the pheromone-emitting sources per unit area or the distribution density of the vapor dispensers.in the field. Accordingly, labor saving is desirable in the field work of setting the vapor dispensers of insectan sex pheromone compound over the field by decreasing the number of the dispensers per unit area of the field by using dispensers capable of exhibiting improved performance to maintain a required concentration of the pheromone vapor prolongedly over a length of time.

For example, it is estimated that, in order to maintain an appropriate concentration of an insectan pheromone compound in the atmospheric air over the field assuming that the density of the vapor dispensers is about 1000 per hectare of the field, each vapor dispenser should contain at least 40 mg of the pheromone compound and the dispenser should have a structure suitable to ensure a stable and controlled rate of vapor release of the pheromone compound permeating the dispenser walls over a length of period. The present invention has been completed as a result of the investigations to solve these problems and the dimensions of the dispenser here disclosed including, for example, 0.4 to 3.0 mm of the inner diameter and 0.1 to 2.0 mm of the wall thickness are very critical for the purpose. Further, uniformity of the rate of vapor release of the pheromone compound over a length of period can be ensured only when the tubular dispenser is in a loop-forming configuration of a specific loop diameter and not in a configuration of a straight tube as such or a hook-like or U-shaped bent form.

Following is a presumble reason for the remarkable difference in the performance of vapor dispensers in respect of the uniformity in the rate of vapor release between the loop-forming configuration of the inventive tubular vapor dispensers and the hook-like or U-shaped configuration of the vapor dispensers. In a tubular vapor dispenser, it is of course that the amount of the pheromone compound contained therein is decreased as the compound is released in the form of vapor through the walls leaving a void space in the tube. When the length of a void space in the tube, where the tube walls are not wet with the pheromone compound, exceeds 60 mm, the overall rate of permeation of the pheromone compound through the walls and hence the overall rate of vapor release should be decreased so much. In other words, it is desirable in order to have a uniform rate of vapor release that, however the overall length of the void space in a tube may be long to exceed 60 mm, the void space in the tube is divided into two or more sections in such a manner that the length of any single void section does not exceed 60 mm or the inner walls of the tube are constantly wet with the pheromone compound by the relative movement of the liquid compound in the tube.

When the tubular vapor dispenser is in a loop-forming configuration, the dispenser receives the wind uniformly by virtue of the spreading configuration resulting in uniform vaporization of the pheromone compound over the whole surface of the tubular body so that no void space as a continuum of 60 mm or longer is formed in the tube even when the amount of the pheromone compound left in the tube is smaller than 50% of the initially charged amount. In addition, the loop-forming dispenser tube hanging on a tree twig can rotate adequately by being blown with the wind to increase the chance of the inner walls becoming wet with the pheromone compound.

When the tubular vapor dispenser is in a straight form or in a hook-like or U-shaped bent form, on the contrary, the above mentioned advantages in the inventive vapor dispenser is more or less lost. When a plastic-made tubular vapor dispenser is bonded side-by-side with a metal wire as is taught in U.S. Patent 4,600,146, it may be a possible way that the dispensers are distributed over the field as being deformed in a suitable configuration utilizing the plastic deformability of the metal wire. This method, however, has a problem that the deformed configuration of a large number of dispensers by manual bending works cannot be so uniform as to ensure constancy of the vapor releasing performance from one to the other. On the contrary, the present invention proposes that the tubular vapor dispensers without side-by-side bonding of a metal wire are supplied in a loop-forming configuration worked beforehand in a machine work so that a great improvement can be obtained in the uniformity of the rate of vapor release.

0233109

WHAT IS CLAIMED IS:

1.  A tubular vapor dispenser for sustainedly releasing vapor of an insectan sex pheromone which comprises:
(a) a tubular body made of a plastic resin having a length of at least 120 mm, an inner diameter in the range from 0.4 to 3.0 mm and a wall thickness in the range from 0.1 to 2.0 mm and airtightly sealed at both tube ends; and
(b) an insectan sex pheromone compound in a liquid form capable of permeating the walls of the tubular body and at least partly filling the bore of the tubular body in an amount of at least 40 mg, the tubular body being in a plastically deformed configuration forming at least one loop.

2.  The tubular vapor dispenser as claimed in claim 1 wherein the loop formed by the plastically deformed tubular body has such a dimension that the length of a chord thereof is at least 20 mm as measured between oppositely facing inward portions of the generating lines of the tube surface.

3.  The tubular vapor dispenser as claimed in claim 1 wherein the plastic resin is a polyethylene.

## FIG. 1

## FIG. 2

## FIG. 3